# EUROPÄISCHE PATENTSCHRIFT

(11) **EP 1 344 766 B1**
(45) Veröffentlichungstag und Bekanntmachung des Hinweises auf die Patenterteilung: **07.12.2005**
(21) Anmeldenummer: 03004365.7
(22) Anmeldetag: 03.03.2003
(51) Int. Cl.: C07C 209/78, C07C 263/10, C07C 211/50, C07C 265/14, C08G 18/76

(54) **Verfahren zur Herstellung von Polyisocyanaten der Diphenylmethanreihe mit vermindertem Farbwert**
Process for the preparation of polyisocyanates of the diphenylmethane series having reduced colour
Procédé de préparation de polyisocyanates de la série du diphénylméthane avec couleur atténuée

(30) Priorität: 13.03.2002 DE 10211021
(43) Veröffentlichungstag der Anmeldung: 17.09.2003
(73) Patentinhaber: Bayer MaterialScience AG, 51368 Leverkusen (DE)
(72) Erfinder: Hagen, Torsten, Dr., 40221 Düsseldorf (DE); Kämper, Friedhelm, Dr., 47807 Krefeld (DE); Koch, Daniel, Dr., 47137 Duisburg (DE); Uchdorf, Rudolf, 47829 Krefeld (DE); Wershofen, Stefan, Dr., 41065 Mönchengladbach (DE)

(56) Entgegenhaltungen:
- EP-A- 0 546 398
- US-A- 5 364 958
- US-A- 6 127 463

## Beschreibung

Die Erfindung betrifft ein Verfahren zur Herstellung von Polyaminen der Diphenylmethanreihe sowie ein Verfahren zur Herstellung von Polyisocyanaten der Diphenylmethanreihe mit einem vermindertem Farbwert, welche durch Umsetzung der entsprechenden Polyamine der Diphenylmethanreihe mit Phosgen gewonnen werden.

Unter Polyisocyanaten der Diphenylmethanreihe werden Isocyanate und Isocyanatgemische folgenden Typs verstanden:

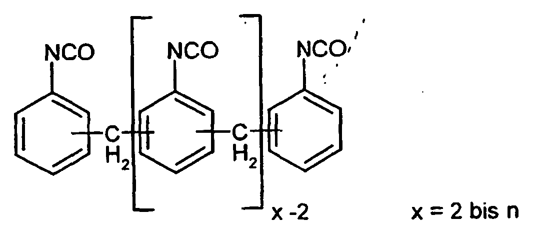

Analog werden unter Polyaminen der Diphenylmethanreihe Verbindungen und Verbindungsgemische folgenden Typs verstanden:

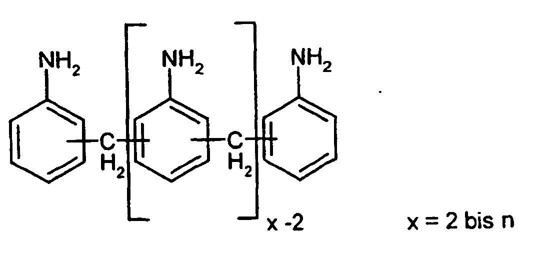

Die großtechnische Herstellung von Isocyanaten durch Umsetzung von Aminen mit Phosgen in Lösungsmitteln ist bekannt und in der Literatur ausführlich beschrieben (Ullmanns Enzyklopädie der technischen Chemie, 4. Auflage, Band 13, Seite 347-357, Verlag Chemie GmbH, Weinheim, 1977). Auf Basis dieses Verfahrens wird das Polyisocyanatgemisch hergestellt, das als Polyisoyanatkomponente bei der Herstellung von Polyurethanschäumen und anderen nach dem Polyadditionsverfahren hergestellten Polyurethankunststoffen dient.

Es ist allgemein bekannt, dass bei diesem Verfahren auch unerwünschte Farbstoffe oder farbgebende Komponenten gebildet werden, die auch bei der Weiterverarbeitung zu Polyurethanschäumen oder anderen Polyurethankunststoffen erhalten bleiben. Obwohl die Eigenfarbe der Polyisocyanat-Polyadditionsprodukte deren mechanische Eigenschaften nicht negativ beeinflusst, werden vom Verbraucher im wesentlichen farblose Produkte gewünscht. Als Maß für die Verfärbung des Polyisocyanates dient die Extinktion bei verschiedenen Wellenlängen.

Daher ist seit längerer Zeit die Reduzierung der Farbwerte von Polyisocyanaten der Diphenylmethanreihe das Ziel zahlreicher Versuche und Arbeiten, die in der Literatur beschrieben sind. So beschreibt beispielsweise DE-A1-4208359 die Behandlung von solchen Isocyanaten mit Wasserstoff in Gegenwart von Trägerkatalysatoren. DE-A1-4232769 beschreibt die Zugabe von Aminen, Harnstoffen und Antioxidantien zum Isocyanat. DE-A1-19815055 beschreibt die Farbverbesserung von Polyisocyanaten der Diphenylmethanreihe durch Bestrahlung mit Licht über einen längeren Zeitraum. DE-A1-19804915 beschreibt die Aufhellung von Polyisocyanaten der Diphenylmethanreihe durch eine komplizierte zeit- und temperaturgestufte Formaldehydzugabe auf der Polyaminstufe, die dann durch Phosgenierung in das gewünschte Isocyanat überführt wird.

Nachteilig an allen diesen Vorgehensweisen ist, dass sie technisch aufwendig sind und/oder die Verwendung isocyanatfremder Hilfsstoffe erfordern oder wenig effizient sind.

Aufgabe der vorliegenden Erfindung war es daher, ein technisch einfaches und sicheres Verfahren bereit zu stellen, mit dem Polyisocyanate der Diphenylmethanreihe mit niedrigen Farbwerten hergestellt werden können. Es war ebenfalls Aufgabe der vorliegenden Erfindung, ein einfaches Verfahren zur Herstellung von Polyaminen der Diphenylmethanreihe bereit zu stellen, aus denen Polyisocyanate der Diphenylmethanreihe mit niedrigen Farbwerten durch Phosgenierung hergestellt werden können.

Die Aufgabe wird erfindungsgemäß gelöst durch ein Verfahren zur Herstellung von Polyaminen der Diphenylmethanreihe, bei dem man
a) Anilin und Formaldehyd in Gegenwart eines sauren Katalysators zu Polyaminen umsetzt und danach
b) das Reaktionsgemisch mit einer Base neutralisiert, nach der Neutralisation die wässrige und die organische Phase trennt und die organische Phase mit einer Base versetzt.

Die Aufgabe wird ebenfalls erfindungsgemäß gelöst durch ein Verfahren zur Herstellung von Polyisocyanaten der Diphenylmethanreihe, bei dem man
a) Anilin und Formaldehyd in Gegenwart eines sauren Katalysators zu Polyaminen umsetzt und danach
b) das Reaktionsgemisch mit einer Base neutralisiert, nach der Neutralisation die wässrige und die organische Phase trennt und die organische Phase mit einer Base versetzt und danach
c) die Polyamine durch Phosgenierung zu den entsprechenden Polyisocyanaten umsetzt.

Das erfindungsgemäße Verfahren kann sowohl kontinuierlich als auch diskontinuierlich durchgeführt werden.

Mit dem erfindungsgemäßen Verfahren können Polyisocyanate mit niedrigen Farbwerten hergestellt werden. Als Farbwert wird hierbei die gemessene Extinktion einer Lösung von Polyisocyanat in Monochlorbenzol, enthaltend 2 Gew.-% Polyisocyanat, bei einer Schichtdicke von 10 mm und Raumtemperatur gegen Monochlorbenzol bei definierten Wellenlängen verstanden.

Das im erfindungsgemäßen Verfahren eingesetzte Polyamin bzw. Polyamingemisch der Diphenylmethanreihe wird durch Kondensation von Anilin und Formaldehyd in Gegenwart eines sauren Katalysators erhalten (H. J. Twitchett, Chem. Soc. Rev. 3(2), 209 (1974), W. M. Moore in: Kirk-Othmer Encycl. Chem. Technol., 3^{rd}. Ed., New York, 2, 338-348 (1978)). Für das erfindungsgemäße Verfahren ist es dabei unerheblich, ob Anilin und Formaldehyd zunächst in Abwesenheit des sauren Katalysators vermischt werden und der saure Katalysator anschließend zugesetzt wird oder ob ein Gemisch aus Anilin und saurem Katalysator mit Formaldehyd zu Reaktion gebracht wird.

Geeignete Polyamingemische der Diphenylmethanreihe werden üblicherweise erhalten durch Kondensation von Anilin und Formaldehyd im Stoffmengenverhältnis (molares Verhältnis) 20 bis 1,6, bevorzugt 10 bis 1,8 sowie einem Stoffmengenverhältnis (molares Verhältnis) von Anilin und saurem Katalysator von 20 bis 1, bevorzugt 10 bis 2.

Formaldehyd wird technisch üblicherweise als wässrige Lösung eingesetzt. Es können jedoch auch andere methylengruppenliefernde Verbindungen wie z.B. Polyoxymethylenglykol, para-Formaldehyd oder Trioxan eingesetzt werden.

Als saure Katalysatoren haben sich starke organische und vorzugsweise anorganische Säuren bewährt. Geeignete Säuren sind beispielsweise Salzsäure, Schwefelsäure, Phosphorsäure und Methansulfonsäure. Bevorzugt wird Salzsäure eingesetzt.

In einer bevorzugten Ausführungsform des Verfahrens wird Anilin und saurer Katalysator zunächst vermischt. Dieses Gemisch wird, ggf. nach Abfuhr von Wärme, in einem weiteren Schritt mit Formaldehyd bei Temperaturen zwischen 20°C und 100°C, bevorzugt bei 30°C bis 70°C in geeigneter Weise vermischt und anschließend in einem geeigneten Verweilzeitapparat einer Vorreaktion unterzogen. Die Vorreaktion erfolgt bei Temperaturen zwischen 20°C bis 100°C, vorzugsweise im Temperaturbereich 30°C bis 80°C. Im Anschluss an Vermischung und Vorreaktion wird die Temperatur des Reaktionsgemisches in Stufen oder kontinuierlich und ggf. unter Überdruck auf eine Temperatur von 100°C bis 250°C, bevorzugt auf 100°C bis 180°C, besonders bevorzugt auf eine Temperatur von 100°C bis 160°C gebracht.

Es ist jedoch ebenfalls möglich, in einer anderen Ausführungsform des Verfahrens Anilin und Formaldehyd zunächst in Abwesenheit des sauren Katalysators im Temperaturbereich von 5°C bis 130°C, bevorzugt von 40°C bis 100°C, besonders bevorzugt von 60°C bis 90°C, zu vermischen und zur Reaktion zu bringen. Hierbei bilden sich Kondensationsprodukte aus Anilin und Formaldehyd (sog. Aminal). Im Anschluss an die Aminalbildung kann im Reaktionsgemisch vorhandenes Wasser durch Phasentrennung oder andere geeignete Verfahrensschritte, beispielsweise durch Destillation, entfernt werden. Das Kondensationsprodukt wird dann in einem weiteren Verfahrensschritt mit dem sauren Katalysator in geeigneter Weise vermischt und in einem Verweilzeitapparat bei 20°C bis 100°C, bevorzugt 30°C bis 80°C einer Vorreaktion unterzogen. Anschließend wird die Temperatur des Reaktionsgemisches in Stufen oder kontinuierlich und ggf. unter Überdruck auf eine Temperatur von 100°C bis 250°C, bevorzugt auf 100°C bis 180°C, besonders bevorzugt auf eine Temperatur von 100°C bis 160°C gebracht.

Die Umsetzung von Anilin und Formaldehyd in Gegenwart eines sauren Katalysators zu Polyaminen der Diphenylmethanreihe kann in Anwesenheit weiterer Stoffe (z.B. Lösungsmittel, Salze, organische und anorganische Säuren) geschehen.

Zur Aufarbeitung des sauren Reaktionsgemisches wird das Reaktionsgemisch nach dem Stand der Technik mit einer Base neutralisiert. Nach dem Stand der Technik erfolgt die Neutralisation üblicherweise bei Temperaturen von beispielsweise 90 bis 100°C (H.J. Twitchett, Chem. Soc. Rev. 3(2), 223 (1974)). Als Basen sind beispielsweise geeignet die Hydroxide der Alkali- und Erdalkalielemente. Vorzugsweise kommt wässrige NaOH zur Anwendung.

Die Neutralisation erfolgt beispielsweise in der Weise, dass das saure Reaktionsgemisch der Anilin/Formaldehydkondensation mit der Base vermischt und einem Verweilzeitapparat (beispielsweise Rührkessel, Rührkesselkaskade, Strömungsrohr, Umpumpreaktor) zugeführt wird. Bei geeigneten Verweilzeitapparaten (z.B. Rührkessel) kann die Vermischung von saurem Kondensationsgemisch und Base auch direkt im Verweilzeitapparat erfolgen.

Im Anschluss an die Neutralisation wird nach dem Stand der Technik die organische von der wässrigen Phase durch geeignete Verfahren (z.B. Phasentrennung in einer Scheideflasche) getrennt. Diese Trennung von organischer und wässriger Phase kann bei der selben Temperatur erfolgen, bei der die Neutralisation des sauren Umlagerungsgemisches erfolgte. Die nach Abtrennung der wässrigen Phase verbleibende, Produkt enthaltende organische Phase wird nach dem Stand der Technik zur Abtrennung von Salzen und überschüssiger Base einer Wäsche unterzogen. Anschließend wird die gereinigte organische Phase von überschüssigem Anilin und anderen im Gemisch vorhandenen Stoffen (z.B. weiteren Lösungsmitteln) durch geeignete physikalische Trennverfahren wie z.B. Destillation, Extraktion oder Kristallisation befreit.

In dem erfindungsgemäßen Verfahren führt man nach der Neutralisation einen zusätzlichen Verfahrensschritt durch, in dem die organische Phase mit einer Base behandelt wird. Dadurch erreicht man, dass die nach der Phosgenierung erhaltenen Polyisocyanate einen verminderten Farbwert aufweisen.

Die Neutralisation wird im erfindungsgemäßen Verfahren beispielsweise bei einer dem Stand der Technik entsprechenden Temperatur, bevorzugt bei 90°C bis 100°C, durchgeführt. Anschließend werden die wässrige und die organische Phase nach einer der üblichen Methoden (beispielsweise durch Phasentrennung in einer Scheideflasche) getrennt. Nach der Phasentrennung wird die organische Phase der neutralisierten Reaktionsmischung in einem Verweilzeitapparat mit einer Base, beispielsweise mit Hydroxiden der Alkali- oder Erdalkalielementen oder deren wässrigen Lösungen, bevorzugt mit wässriger Natronlauge, behandelt.

Die Behandlung der organischen Phase der neutralisierten Reaktionsmischung mit der Base erfolgt bevorzugt bei Temperaturen von oberhalb 60°C, besonders bevorzugt 61 °C bis 300°C, ganz besonders bevorzugt 80°C bis 200°C, insbesondere ganz besonders bevorzugt 90°C bis 160°C.

Zur Einstellung der Temperatur für die Behandlung der organischen Phase der neutralisierten Reaktionsmischung mit der Base kann Zufuhr oder Abfuhr von Wärme notwendig sein. Dies richtet sich insbesondere nach der gewünschten Temperatur, bei welcher die Behandlung der organischen Phase mit der Base erfolgen soll, aber auch nach der Temperatur der eingesetzten organischen Phase und der Temperatur der eingesetzten Base bzw. Basenlösung. Um das Sieden unterhalb einer gewünschten Temperatur zu verhindern, muss das Verfahren gegebenenfalls bei erhöhtem Druck durchgeführt werden.

Die zur Behandlung der organischen Phase verwendete Base wird bevorzugt in Mengen von größer 1 %, besonders bevorzugt 2 - 400%, ganz besonders bevorzugt 5 bis 150 % der für die Neutralisation des für die Kondensationsreaktion verwendeten sauren Katalysators stöchiometrisch benötigten Menge eingesetzt. Die organische Phase wird in Gegenwart der Base bevorzugt für eine Verweilzeit von ≥ 0,1 min, besonders bevorzugt 0,1 bis 180 min, ganz besonders bevorzugt 2 bis 150 min, insbesondere ganz besonders bevorzugt 10 bis 120 min auf einer Temperatur von oberhalb 60°C gehalten. Die Behandlung der organischen Phase des neutralisierten Reaktionsgemisches mit der Base erfolgt dabei beispielsweise in der Weise, dass die organische Phase mit der Base vermischt und einem Verweilzeitapparat zugeführt wird (beispielsweise Rührkessel, Rührkesselkaskade, Strömungsrohr oder Umpumpreaktor). Bei geeigneten Verweilzeitapparaten kann die Vermischung von organischer Phase und Base auch direkt im Verweilzeitapparat erfolgen.

Der Effekt auf die Farbe der Polyisocyanate der Diphenylmethanreihe wird verstärkt, wenn in dem Verweilzeitapparat für eine ausreichende Durchmischung der organischen und wässrigen Phase gesorgt wird. Dies kann durch Anwendung der in der Technik bekannten Methoden, beispielsweise durch statische oder dynamische Mischer oder Erzeugung von Turbulenz erfolgen. Nach der Behandlung der organischen Phase mit Base wird, gegebenenfalls nach Zugabe von Wasser, eine Phasentrennung vorgenommen und die organische Phase den weiteren Aufarbeitungsschritten (z.B. Wäsche und Destillation) zugeführt und so ein Polyamin der Diphenylmethanreihe erhalten. Die NaOH-haltige wässrige Phase aus der Phasentrennung kann vorteilhaft in die Neutralisationsstufe des Verfahrens eingebracht werden, gegebenenfalls nach Zugabe von Wasser und/oder NaOH, um die gewünschte Basenmenge bzw. -konzentration einzustellen.

Das so erhaltene Polyamin der Diphenylmethanreihe (Roh-MDA) wird nach den bekannten Methoden mit Phosgen in einem inerten organischen Lösungsmittel zu den entsprechenden Isocyanaten umgesetzt. Das Molverhältnis von Roh-MDA zu Phosgen wird zweckmäßigerweise so bemessen, dass pro Mol NH₂-Gruppe 1 bis 10 mol, vorzugsweise 1,3 bis 4 mol Phosgen in der Reaktionsmischung vorliegen. Als inerte Lösungsmittel haben sich chlorierte, aromatische Kohlenwasserstoffe wie z.B. Monochlorbenzol, Dichlorbenzole, Trichlorbenzole, die entsprechenden Toluole und Xylole sowie Chlorethylbenzol bewährt. Insbesondere Anwendung finden als inerte organische Lösungsmittel Monochlorbenzol, Dichlorbenzol oder Mischungen dieser Chlorbenzole. Die Menge an Lösungsmittel wird vorzugsweise so bemessen, dass die Reaktionsmischung einen Isocyanatgehalt von 2 bis 40 Gew.-%, vorzugsweise zwischen 5 und 20 Gew.-%, bezogen auf das Gesamtgewicht der Reaktionsmischung, aufweist. Nach beendeter Phosgenierung werden aus der Reaktionsmischung das überschüssige Phosgen, das inerte organische Lösungsmittel, die gebildete HCl oder Mischungen davon durch geeignete Verfahren (z.B. Destillation) abgetrennt.

Das nach dem erfindungsgemäßen Verfahren hergestellte Roh-MDI besitzt eine deutlich reduzierte Färbung. Es lassen sich jedoch noch weitere analytische Unterschiede im hergestellten MDI nachweisen (wie z.B. ein erhöhter Gehalt an Isocyanatgruppen).

### Beispiele:

### Beispiel 1 (Vergleichsbeispiel)

Zu 1000 g Anilin wurden bei 80°C innerhalb von 20 min zugleich 1048,9 g Anilin und 1026,5 g einer 32,2 %igen wässrigen Formaldehydlösung getropft. Nach der Zugabe wurde noch 10 min gerührt und anschließend eine Phasentrennung bei 70°C bis 80°C vorgenommen. Von der organischen Phase wurde eine Menge von 623,3 g auf 35°C temperiert und anschließend bei dieser Temperatur innerhalb von 30 min mit der restlichen organischen Phase und 626,8 g einer 31,9 %igen wässrigen Salzsäure versetzt. Nach beendeter Zugabe und einer 30 minütigen Nachrührzeit bei dieser Temperatur wurde während 10 min auf 60°C aufgeheizt und 30 min bei dieser Temperatur gehalten. Anschließend wurde innerhalb von 30 min auf Rückflusstemperatur aufgeheizt und 10h unter Rückfluss gerührt. Das so erhaltene saure Umlagerungsgemisch wurde anschließend mit 532,4 g einer 49,6 %igen wässrigen Natronlauge und 730 ml siedendem Wasser versetzt. Nach weiteren 15 min Rühren unter Rückfluss bei ca. 100°C wurde bei 80°C bis 90°C eine Phasentrennung vorgenommen und die organische Phase weitere 2x mit je 1000 ml siedendem Wasser gewaschen. Die organische Phase wurde anschließend unter vermindertem Druck vom überschüssigen Anilin befreit. Von dem so erhaltenen Polyamin wurden 50 g in 255 ml Chlorbenzol gelöst, auf 55°C erwärmt und innerhalb von 10s unter intensivem Rühren zu einer auf 0°C temperierten Lösung von 105 g Phosgen in 310 ml Chlorbenzol gegeben. Die Suspension wurde unter Durchleiten von Phosgen innerhalb von 45 min auf 100°C und anschließend während 10 min auf Rückflusstemperatur aufgeheizt. Nach weiteren 10 min bei dieser Temperatur wurde das Lösungsmittel unter vermindertem Druck bis zu einer Sumpftemperatur von 100°C abdestilliert. Das rohe Isocyanat wurde anschließend in einer Destillationsapparatur bei einem Druck von 4 bis 6 mbar durch ein auf 260°C erwärmtes Heizbad bis zum ersten Produktübergang erhitzt und daraufhin innerhalb von 5 min auf Raumtemperatur abgekühlt. Von dem so erhaltenen Isocyanat wurde 1,0 g in Chlorbenzol gelöst und mit Chlorbenzol zu 50 ml verdünnt. Die so erhaltene Lösung besaß bei einer Wellenlänge von 430 nm, einer Schichtdicke von 10 mm und Raumtemperatur eine Extinktion von 0,191 gegen Chlorbenzol.

### Beispiel 2 (erfindungsgemäß)

Analog Beispiel 1 wurde mit den dort angegebenen Mengen an Anilin, wässriger Formaldehydlösung und wässriger Salzsäure ein saures Umlagerungsgemisch hergestellt und anschließend mit 532,4 g 49,6 %iger wässriger Natronlauge und 730 ml Wasser bei ca. 100°C neutralisiert. Die Phasen wurden getrennt und die organische Phase mit 1477 g einer 50 %iger wässriger Natronlauge unter Rühren 90 min bei ca. 140°C zum Sieden erhitzt. Nach einer erneuten Phasentrennung wurde die organische Phase gemäß Beispiel 1 durch Waschen mit Wasser und Destillieren unter vermindertem Druck weiter gereinigt und das resultierende Polyamin der Diphenylmethanreihe mit Phosgen in das entsprechende Polyisocyanat überführt. Die Extinktion bei 430 nm gemäß der in Beispiel 1 beschriebenen Methode betrug 0,133 gegen Chlorbenzol.

### Beispiel 3 (Vergleichsbeispiel)

1226 g der nach Neutralisation eines sauren Umlagerungsgemisches erhaltenen organischen Phase aus der großtechnischen Produktion von Polyaminen der Diphenylmethanreihe wurden analog Beispiel 1 durch dreimaliges Waschen mit Wasser und durch Destillieren unter vermindertem Druck aufgearbeitet. Das resultierende Polyamin der Diphenylmethanreihe wurde mit Phosgen in das entsprechende Polyisocyanat überführt. Die Extinktion bei 430 nm gemäß der in Beispiel 1 beschriebenen Methode betrug 0,114 gegen Chlorbenzol.

### Beispiel 4 (erfindungsgemäß)

1226 g der in Bespiel 3 genannten, nach Neutralisation eines sauren Umlagerungsgemisches erhaltenen organischen Phase aus der großtechnischen Produktion von Polyamin der Diphenylmethanreihe wurden mit 368 g 50 %iger wässriger Natronlauge unter Rühren 2 h bei ca. 130°C zum Sieden erhitzt. Nach einer erneuten Phasentrennung wurde die organische Phase gemäß Beispiel 3 durch Waschen mit Wasser und Destillieren unter vermindertem Druck weiter gereinigt und das resultierende Polyamin der Diphenylmethanreihe mit Phosgen in das entsprechende Polyisocyanat überführt. Die Extinktion bei 430 nm gemäß der in Beispiel 1 beschriebenen Methode betrug 0,084 gegen Chlorbenzol.

## Patentansprüche

1. Verfahren zur Herstellung von Polyaminen der Diphenylmethanreihe, bei dem man
a) Anilin und Formaldehyd in Gegenwart eines sauren Katalysators zu Polyaminen umsetzt und danach
b) das Reaktionsgemisch mit einer Base neutralisiert, nach der Neutralisation die wässrige und die organische Phase trennt und die organische Phase mit einer Base versetzt.

2. Verfahren nach Anspruch 1, bei dem man als Base Hydroxide der Alkali- oder Erdalkalielemente oder deren wässrige Lösungen einsetzt.

3. Verfahren nach Anspruch 1 oder 2, bei dem man als Base wässrige NaOH einsetzt.

4. Verfahren zur Herstellung von Polyisocyanaten der Diphenylmethanreihe, bei dem man
a) Anilin und Formaldehyd in Gegenwart eines sauren Katalysators zu Polyaminen umsetzt und danach
b) das Reaktionsgemisch mit einer Base neutralisiert, nach der Neutralisation die wässrige und die organische Phase trennt und die organische Phase mit einer Base versetzt und danach
c) die Polyamine durch Phosgenierung zu den entsprechenden Polyisocyanaten umsetzt.

5. Verfahren nach Anspruch 4, bei dem man als Base Hydroxide der Alkali- oder Erdalkalielemente oder deren wässrige Lösungen einsetzt.

6. Verfahren nach Anspruch 4 oder 5, bei dem man als Base wässrige NaOH einsetzt.

## Claims

1. Process for producing polyamines of the diphenyl methane series, wherein
a) aniline and formaldehyde are reacted in the presence of an acid catalyst to form polyamines and then
b) the reaction mixture is neutralised with a base, the aqueous and the organic phases are separated after neutralisation and a base is added to the organic phase.

2. Process according to claim 1, wherein hydroxides of the alkali or alkaline-earth metal elements or aqueous solutions thereof are used as a base.

3. Process according to claim 1 or 2, wherein aqueous NaOH is used as a base.

4. Process for producing polyisocyanates of the diphenyl methane series, wherein
a) aniline and formaldehyde are reacted in the presence of an acid catalyst to form polyamines and then
b) the reaction mixture is neutralised with a base, the aqueous and the organic phases are separated after neutralisation and a base is added to the organic phase and then
c) the polyamines are reacted to form the corresponding polyisocyanates by phosgenation.

5. Process according to claim 4, wherein hydroxides of the alkali or alkaline-earth metal elements or aqueous solutions thereof are used as a base.

6. Process according to claim 4 or 5, wherein aqueous NaOH is used as a base.

## Revendications

1. Procédé de préparation de polyamines de la série du diphénylméthane, dans lequel
a) on transforme de l'aniline et de l'aldéhyde formique en polyamines en présence d'un catalyseur acide et
b) on neutralise ensuite le mélange réactionnel avec une base, on sépare la phase aqueuse et la phase organique après la neutralisation et on mélange la phase organique avec une base.

2. Procédé selon la revendication 1, dans lequel on utilise comme base des hydroxydes d'éléments alcalins ou alcalino-terreux ou des solutions aqueuses de ceux-ci.

3. Procédé selon la revendication 1 ou 2, dans lequel on utilise comme base du NaOH aqueux.

4. Procédé de préparation de polyisocyanates de la série du diphénylméthane, dans lequel
a) on transforme de l'aniline et de l'aldéhyde formique en polyamines en présence d'un catalyseur acide,
b) on neutralise ensuite le mélange réactionnel avec une base, on sépare la phase aqueuse et la phase organique après la neutralisation et on mélange la phase organique avec une base et
c) on transforme ensuite les polyamines en polyisocyanates correspondants par phosgénation.

5. Procédé selon la revendication 4, dans lequel on utilise comme base des hydroxydes d'éléments alcalins ou alcalino-terreux ou des solutions aqueuses de ceux-ci.

6. Procédé selon la revendication 4 ou 5, dans lequel on utilise comme base du NaOH aqueux.
